# DEMANDE DE BREVET EUROPEEN

(11) **EP 1 935 401 A2**
(43) Date de publication de la demande: **25.06.2008**
(21) Numéro de dépôt: 07123273.0
(22) Date de dépôt: 14.12.2007
(51) Int. Cl.: A61K 8/89, A61Q 5/06

(54) **Procédé de traitement des fibres capillaires avec des compositions contenant des siliconés reactives**

(30) Priorité: 20.12.2006 FR 0655728
(71) Demandeur: L'ORÉAL, 75008 Paris (FR)
(72) Inventeur: Benabdillah, Katarina, 95130, LE PLESSIS-BOUCHARD (FR)
(74) Mandataire: Bourdeau, Françoise

(57) **Abrégé**

Procédé de traitement des fibres capillaires comprenant les étapes suivantes :
- application sur les fibres kératiniques d'au moins un composé X et au moins un composé Y, l'un au moins des composés X et Y étant un composé siliconé, lesdits composés X et Y étant susceptibles de réagir ensemble par une réaction d'hydrosilylation, de condensation, ou de réticulation en présence de peroxyde lorsqu'ils sont mis en contact l'un avec l'autre ;
- élévation de la température des fibres kératiniques, au moyen d'un fer chauffant, à une température au moins égale à 60°C, l'élévation de la température étant effectuée avant ou après un rinçage éventuel des fibres kératiniques ;

le ou les composés X, le ou les composés Y, étant appliqués sur les fibres kératiniques à partir de plusieurs compositions contenant le ou les composés X, le ou les composés Y, seuls ou en mélange, ou à partir d'une seule composition contenant le ou les composés X, le ou les composés Y.

## Description

L'invention concerne un procédé de traitement des fibres capillaires, et l'utilisation dudit procédé pour améliorer le lissage des fibres capillaires.

De nombreux procédés de traitement des cheveux utilisent des composés siliconés. Il est en effet connu que les composés siliconés sont des actifs cosmétiques qui améliorent les propriétés cosmétiques des cheveux. Ils ont un effet conditionneur sur les cheveux et apportent du lissage.

Cependant, cet apport de brillance apporté par les silicones a tendance à s'estomper rapidement avec le temps.

Par ailleurs, de nombreux procédés de traitement des cheveux utilisent une étape de chauffage des cheveux. Cependant, le chauffage des cheveux peut avoir un effet dégradant sur les fibres, et surtout les résultats ne sont pas durables.

Ainsi, la demande de brevet WO 99/17719 divulgue un procédé de traitement des cheveux, pour le conditionnement et la mise en forme non permanente des cheveux. Ce procédé comprend l'application d'une composition non rincée comprenant un agent conditionnant siliconé non volatil, une résine et un vecteur, puis l'utilisation d'un appareil chauffant pour sécher ou coiffer les cheveux, cette opération induisant une réduction du module de flexion d'au moins 1%. L'agent conditionnant siliconé non volatil représente de 0,1 à 2% en poids du poids total de la composition.

La demande de brevet EP 1 582 198 décrit un procédé de traitement des fibres capillaires comprenant une étape d'application sur les fibres capillaires d'une composition comprenant au moins 5% en poids, par rapport au poids total de la composition, d'au moins une silicone choisie parmi les silicones arylées et les gommes de silicone, puis une étape d'élévation de la température des fibres capillaires, au moyen d'un fer chauffant, à une température comprise entre 150 et 250°C.

Le but de la présente invention est donc de fournir un procédé de traitement des fibres capillaires qui remédie aux inconvénients de l'art antérieur.

En particulier, la présente invention a pour but de fournir un procédé de traitement des fibres capillaires qui permet de conférer aux fibres un bon niveau de lissage sans reprise de frisottis et qui permet de prolonger cet effet dans le temps, avec une dégradation limitée des fibres. Par ailleurs, l'effet résiste mieux aux shampooings.

Ledit procédé doit en outre permettre d'augmenter la raideur et améliorer le lissage des fibres capillaires.

La Demanderesse a trouvé qu'il était possible de remédier aux inconvénients de l'art antérieur et de remplir les objectifs précités en mettant en oeuvre un procédé de traitement des fibres capillaires comprenant une étape d'application sur les fibres capillaires d'au moins un composé X et au moins un composé Y, l'un au moins des composés X et Y étant un composé siliconé, lesdits composés X et Y étant susceptibles de réagir ensemble par une réaction d'hydrosilylation, de condensation, ou de réticulation en présence de peroxyde lorsqu'ils sont mis en contact l'un avec l'autre, et une étape d'élévation de la température des fibres capillaires, au moyen d'un fer chauffant, à une température au moins égale à 60°C, l'élévation de la température étant effectuée avant ou après un rinçage éventuel des fibres kératiniques.

Ainsi, l'invention a pour objet un procédé de traitement des fibres capillaires comprenant les étapes suivantes :
- application sur les fibres kératiniques d'au moins un composé X et au moins un composé Y, l'un au moins des composés X et Y étant un composé siliconé, lesdits composés X et Y étant susceptibles de réagir ensemble par une réaction d'hydrosilylation, de condensation, ou de réticulation en présence de peroxyde lorsqu'ils sont mis en contact l'un avec l'autre ;
- élévation de la température des fibres kératiniques, au moyen d'un fer chauffant, à une température au moins égale à 60°C, l'élévation de la température étant effectuée avant ou après un rinçage éventuel des fibres kératiniques.

Le ou les composés X, le ou les composés Y, peuvent être appliqués sur les fibres kératiniques à partir de plusieurs compositions contenant le ou les composés X, le ou les composés Y, seuls ou en mélange, ou à partir d'une seule composition contenant le ou les composés X, le ou les composés Y.

### Composés X et Y

Par composé siliconé, on entend un composé comprenant au moins deux unités organosiloxanes. Selon un mode de réalisation particulier, les composés X et les composés Y sont siliconés. Les composés X et Y peuvent être aminés ou non aminés. Ils peuvent comprendre des groupes polaires pouvant être choisis parmi les groupes suivants -COOH, -COO- , -COO- , -OH , -NH₂, -NH-,-NR-, -SO₃H, -SO₃⁻,-OCH₂CH₂-, -O-CH₂CH₂CH₂-, -O-CH₂CH(CH₃)-, -NR₃⁺, -SH, -NO₂, I, CI, Br, -CN, -PO₄³⁻, -CONH- , -CONR-, -CONH₂, -CSNH-, -SO₂-, -SO-, -SO₂NH-, -NHCO- , -NHSO₂-,-NHCOO-, -OCONH-, -NHCSO- et -OCSNH-, R représentant un groupe alkyle.

Selon un autre mode de réalisation, au moins un des composés X et Y est un polymère dont la chaîne principale est formée majoritairement d'unités organosiloxanes.

Parmi les composés siliconés cités ci-après, certains peuvent présenter à la fois des propriétés filmogènes et adhésives, selon par exemple leur proportion en silicone ou selon qu'on les utilise en mélange avec un additif particulier. Il est par conséquent possible de moduler les propriétés filmogènes ou les propriétés adhésives de tels composés selon l'utilisation envisagée, c'est en particulier le cas pour les silicones élastomères réactives dites "room temperature vulcanization".

Les composés X et Y peuvent réagir ensemble à une température variant entre la température ambiante et 180 °C. Avantageusement, les composés X et Y sont susceptibles de réagir ensemble à température ambiante (20 ± 5 °C) et pression atmosphérique, avantageusement en présence d'un catalyseur, par une réaction d'hydrosilylation ou une réaction de condensation, ou une réaction de réticulation en présence d'un peroxyde.

### 1- Composés X et Y susceptibles de réagir par hydrosilylation

Selon un mode de réalisation, les composés X et Y sont susceptibles de réagir ensemble par hydrosilylation, cette réaction pouvant être de manière simplifiée schématisée comme suit : 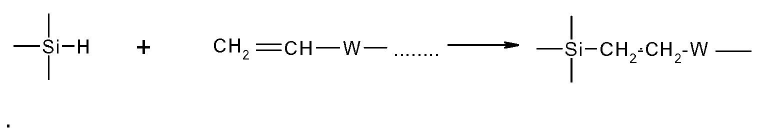 avec W représentant une chaîne carbonée et/ou siliconée contenant un ou plusieurs groupements aliphatiques insaturés.

Dans ce cas, le composé X peut être choisi parmi les composés siliconés comprenant au moins deux groupements aliphatiques insaturés. A titre d'exemple, le composé X peut comprendre une chaîne principale siliconée dont les groupements aliphatiques insaturés sont pendants à la chaîne principale (groupe latéral) ou situés aux extrémités de la chaîne principale du composé (groupe terminal). On appellera, dans la suite de la description, ces composés particuliers des polyorganosiloxanes à groupements aliphatiques insaturés.

Selon un mode de réalisation, le composé X est choisi parmi les polyorganosiloxanes comprenant au moins deux groupements aliphatiques insaturés, par exemple deux ou trois groupements vinyliques ou allyliques, liés chacun à un atome de silicium.

Selon un mode de réalisation avantageux, le composé X est choisi parmi les polyorganosiloxanes comprenant des unités siloxanes de formule : 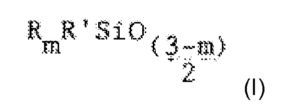 dans laquelle :
- R représente un groupe hydrocarboné monovalent, linéaire ou cyclique, comprenant de 1 à 30 atomes de carbone, de préférence de 1 à 20, et mieux de 1 à 10 atomes de carbone, comme par exemple un radical alkyle à chaîne courte, comprenant par exemple de 1 à 10 atomes de carbone, en particulier un radical méthyle ou encore un groupement phényle, de préférence un radical méthyle ;
- m est égal à 1 ou 2 ; et
- R' représente :
   - un groupement hydrocarboné aliphatique insaturé comprenant de 2 à 10, de préférence de 2 à 5 atomes de carbone comme par exemple un groupe vinyle ou un groupe -R"-CH=CHR"' dans lequel R" est une chaîne hydrocarbonée aliphatique divalente, comprenant de 1 à 8 atomes de carbone, liée à l'atome de silicium et R"' est un atome d'hydrogène ou un radical alkyle comprenant de 1 à 4 atomes de carbone, de préférence un atome d'hydrogène ; on peut citer comme groupement R' les groupements vinyle, allyle et leurs mélanges ; ou
   - un groupement hydrocarboné cyclique insaturé comprenant de 5 à 8 atomes de carbone comme par exemple un groupe cyclohexényle.

De préférence R' est un groupement hydrocarboné aliphatique insaturé, de préférence un groupe vinyle.

Selon un mode de réalisation particulier, le polyorganosiloxane comprend également des unités de formule : 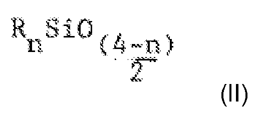 dans laquelle R est un groupe tel que défini plus haut, et n est égal à 1, 2 ou 3.

Selon une variante, le composé X peut être une résine de silicone comprenant au moins deux insaturations éthyléniques, ladite résine étant apte à réagir avec le composé B par hydrosilylation. On peut citer par exemple les résines de type MQ ou MT portant elle-même des extrémités réactives insaturées -CH=CH₂.

Ces résines sont des polymères d'organosiloxanes réticulés.

La nomenclature des résines de silicone est connue sous le nom de "MDTQ", la résine étant décrite en fonction des différentes unités monomériques siloxane qu'elle comprend, chacune des lettres "MDTQ" caractérisant un type d'unité.

La lettre M représente l'unité monofonctionelle de formule (CH₃)₃SiO_{1/2}, l'atome de silicium étant relié à un seul atome d'oxygène dans le polymère comprenant cette unité.

La lettre D signifie une unité difonctionnelle (CH₃)₂SiO_{2/2} dans laquelle l'atome de silicium est relié à deux atomes d'oxygène

La lettre T représente une unité trifonctionnelle de formule (CH₃)SiO_{3/2}.

Dans les motifs M, D, T définis précédemment, au moins un des groupes méthyle peut être substitué par un groupe R différent du groupe méthyle tel qu'un radical hydrocarboné (notamment alkyle) ayant de 2 à 10 atomes de carbone ou un groupe phényle ou bien encore un groupe hydroxyle.

Enfin, la lettre Q signifie une unité tétrafonctionnelle SiO_{4/2} dans laquelle l'atome de silicium est lié à quatre atomes d'hydrogène eux mêmes liés au reste du polymère. Comme exemples de telles résine, on peut citer les résines de silicone MT telles que les poly(phenyl-vinylsilsesquioxane) comme celle commercialisées sous la référence SST-3PV1 par la société Gelest.

De préférence, les composés X comprennent de 0,01 à 1 % en poids de groupes aliphatiques insaturés.

Avantageusement, le composé X est choisi parmi les polyorganopolysiloxanes, notamment ceux comprenant les unités siloxanes (1) et éventuellement (II) décrites précédemment.

Le composé Y comprend de préférence au moins deux groupes Si-H (groupes hydrogénosilanes) libres.

Le composé Y peut être avantageusement choisi parmi les organosiloxanes comprenant au moins une unité alkylhydrogènosiloxane de formule suivante : 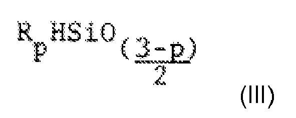 dans laquelle :
R représente un groupe hydrocarboné monovalent, linéaire ou cyclique, comprenant de 1 à 30 atomes de carbone, comme par exemple un radical alkyle ayant de 1 à 30 atomes de carbone, de préférence de 1 à 20 et mieux de 1 à 10 atomes de carbone, en particulier un radical méthyle, ou encore un groupement phényle et p est égal à 1 ou 2. De préférence R est un groupement hydrocarboné, de préférence le méthyle.

Ces composés Y organosiloxanes à unités alkylhydrogènosiloxanes peuvent comprendre en outre des unités de formule : 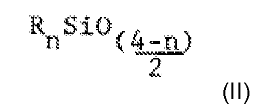 telles que définies plus haut.

Le composé Y peut être une résine de silicone comprenant au moins un motif choisis parmi les motifs M, D, T, Q tels que définis ci-dessus et comprenant au moins un groupe Si-H telles que les poly(methyl-hydridosilsesquioxane) commercialisées sous la référence SST-3MH1.1 par la société Gelest.

De préférence, ces composés Y organosiloxanes comprennent de 0,5 à 2,5 % en poids de groupes Si-H.

Avantageusement, les radicaux R représentent un groupement méthyle dans les formules (I), (II), (III) ci-dessus.

De préférence, ces organosiloxanes Y comprennent des groupes terminaux de formule (CH₃)₃SiO_{1/2}.

Avantageusement, les organosiloxanes Y comprennent au moins deux unités alkylhydrogènosiloxane de formule (H₃C)(H)SiO et comprennent éventuellement des unités (H₃C)₂SiO.

De tels composés Y organosiloxanes à groupements hydrogénosilane sont décrits par exemple dans le document EP 0465744.

Selon une variante, le composé X est choisi parmi les oligomères ou polymères organiques (par organique, on entend des composés dont la chaîne principale est non siliconée, de préférence des composés ne comprenant pas d'atomes de silicium) ou parmi les polymères ou oligomères hybrides organique / silicone, lesdits oligomères ou polymères portant au moins 2 groupements aliphatiques insaturés réactifs, le composé Y étant choisi parmi les hydrogénosiloxanes cités ci-dessus.

Le composé X, de nature organique, peut alors être choisi parmi les polymères ou oligomères vinyliques, (méth)acryliques, les polyesters, les polyuréthanes et / ou les polyurées, les polyéthers, les perfluoropolyéthers, les polyoléfines telles que le polybutène, le polyisobutylène, les dendrimères ou les polymères hyper-ramifiés organiques, ou leurs mélanges.

En particulier, le polymère organique ou la partie organique du polymère hybride peut être choisi parmi les polymères suivants :
a) les polyesters à insaturation(s) éthylénique(s) :
   Il s'agit d'un groupe de polymères de type polyester présentant au moins 2 doubles liaisons éthyléniques, réparties de manière aléatoire dans la chaîne principale du polymère. Ces polyesters insaturés sont obtenus par polycondensation d'un mélange :
      - de diacides carboxyliques aliphatiques linéaires ou ramifiés ou cycloaliphatiques comportant notamment de 3 à 50 atomes de carbone, de préférence de 3 à 20 et mieux de 3 à 10 atomes de carbone, tels que l'acide adipique ou l'acide sébacique, de diacides carboxyliques aromatiques ayant notamment de 8 à 50 atomes de carbone, de préférence de 8 à 20 et mieux de 8 à 14 atomes de carbone, tels que les acides phtaliques, notamment l'acide téréphtalique, et/ou de diacides carboxyliques issus de dimères d'acides gras à insaturations éthyléniques tels que les dimères des acides oléique ou linoléique décrits dans la demande EP-A-959 066 (paragraphe [0021]) commercialisés sous les dénominations Pripol^{®} par la société Unichema ou Empol^{®} par la société Henkel, tous ces diacides devant être exempts de doubles liaisons éthyléniques polymérisables,
      - de diols aliphatiques linéaires ou ramifiés ou cycloaliphatiques comportant notamment de 2 à 50 atomes de carbone, de préférence de 2 à 20 et mieux de 2 à 10 atomes de carbone, tels que l'éthylèneglycol, le diéthylèneglycol, le propylèneglycol, le 1,4-butanediol ou le cyclohexanediméthanol, de diols aromatiques ayant de 6 à 50 atomes de carbone, de préférence de 6 à 20 et mieux de 6 à 15 atomes de carbone tel que le le bisphénol A et le bisphénol B, et/ou de dimères diols issus de la réduction des dimères d'acides gras tels que définis précédemment, et
      - d'un ou de plusieurs diacides carboxyliques ou leurs anhydrides comportant au moins une double liaison éthylénique polymérisable et ayant de 3 à 50 atomes de carbone, de préférence de 3 à 20 et mieux de 3 à 10 atomes de carbone, tels que l'acide maléique, l'acide fumarique ou l'acide itaconique.
b) les polyesters à groupes (méth)acrylate latéraux et / ou terminaux :
   II s'agit d'un groupe de polymères de type polyester obtenus par polycondensation d'un mélange :
      - de diacides carboxyliques aliphatiques linéaires ou ramifiés ou cycloaliphatiques comportant notamment de 3 à 50 atomes de carbone, de préférence de 3 à 20 et mieux de 3 à 10 atomes de carbone, tels que l'acide adipique ou l'acide sébacique, de diacides carboxyliques aromatiques ayant notamment de 8 à 50 atomes de carbone, de préférence de 8 à 20 et mieux de 8 à 14 atomes de carbone, tels que les acides phtaliques, notamment l'acide téréphtalique, et/ou de diacides carboxyliques issus de dimères d'acides gras à insaturation éthyléniques tels que les dimères des acides oléique ou linoléique décrits dans la demande EP-A-959 066 (paragraphe [0021]) commercialisés sous les dénominations Pripol^{®} par la société Unichema ou Empol^{®} par la société Henkel, tous ces diacides devant être exempts de doubles liaisons éthyléniques polymérisables,
      - de diols aliphatiques linéaires ou ramifiés ou cycloaliphatiques comportant notamment de 2 à 50 atomes de carbone, de préférence de 2 à 20 et mieux de 2 à 10 atomes de carbone, tels que l'éthylèneglycol, le diéthylèneglycol, le propylèneglycol, le 1,4-butanediol ou le cyclohexanediméthanol, de diols aromatiques ayant de 6 à 50 atomes de carbone, de préférence de 6 à 20 et mieux de 6 à 15 atomes de carbone tel que le bisphénol A et le bisphénol B, et
      - d'au moins un ester d'acide (méth)acrylique et d'un diol ou polyol ayant de 2 à 20 atomes de carbone, de préférence de 2 à 6 atomes de carbone, tels que le (méth)acrylate de 2-hydroxyéthyle, le (méth)acrylate de 2-hydroxypropyle et le méthacrylate de glycérol.
   Ces polyesters diffèrent de ceux décrits ci-dessus sous le point a) par le fait que les doubles liaisons éthyléniques ne sont pas situées dans la chaîne principale mais sur des groupes latéraux ou à l'extrémité des chaînes. Ces doubles liaisons éthyléniques sont celles des groupes (méth)acrylate présents dans le polymère.
   De tels polyesters sont commercialisés par exemple par la société UCB sous les dénominations EBECRYL^{®} (EBECRYL^{®} 450 : masse molaire 1600, en moyenne 6 fonctions acrylate par molécule, EBECRYL^{®} 652 : masse molaire 1500, en moyenne 6 fonctions acrylate par molécule, EBECRYL^{®} 800 : masse molaire 780, en moyenne 4 fonctions acrylate par molécule, EBECRYL^{®} 810 : masse molaire 1000, en moyenne 4 fonctions acrylate par molécule, EBECRYL^{®} 50 000 : masse molaire 1500, en moyenne 6 fonctions acrylate par molécule).
c) les polyuréthannes et / ou polyurées à groupes (méth)acrylate, obtenus par polycondensation :
   - de diisocyanates, triisocyanates et / ou polyisocyanates aliphatiques cycloaliphatiques et/ou aromatiques ayant notamment de 4 à 50, de préférence de 4 à 30 atomes de carbone, tels que l'hexaméthylènediisocyanate, l'isophoronediisocyanate, le toluènediisocyanate, le diphénylméthanediisocyanate ou les isocyanurates de formule : 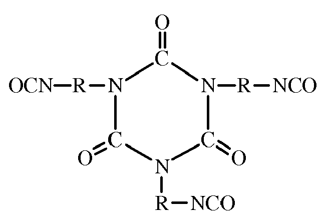 résultant de la trimérisation de 3 molécules de diisocyanates OCN-R-CNO, où R est un radical hydrocarboné linéaire, ramifié ou cyclique comportant de 2 à 30 atomes de carbone ;
   - de polyols, notamment de diols, exempts d'insaturations éthyléniques polymérisables, tels que le 1,4-butanediol, l'éthylèneglycol ou le triméthylolpropane, et / ou de polyamines, notamment de diamines, aliphatiques, cycloaliphatiques et / ou aromatiques ayant notamment de 3 à 50 atomes de carbone, telles que l'éthylènediamine ou l'hexaméthylènediamine, et
   - d'au moins un ester d'acide (méth)acrylique et d'un diol ou polyol ayant de 2 à 20 atomes de carbone, de préférence de 2 à 6 atomes de carbone, tels que le (méth)acrylate de 2-hydroxyéthyle, le (méth)acrylate de 2-hydroxypropyle et le méthacrylate de glycérol.
   De tels polyuréthannes / polyurées à groupes acrylates sont commercialisés par exemple sous la dénomination SR 368 (tris(2-hydroxyéthyl)isocyanurate-triacrylate) ou CRAYNOR^{®} 435 par la société CRAY VALLEY, ou sous la dénomination EBECRYL^{®} par la société UCB (EBECRYL^{®} 210 : masse molaire 1500, 2 fonctions acrylate par molécule, EBECRYL® 230 : masse molaire 5000, 2 fonctions acrylate par molécule, EBECRYL^{®} 270 : masse molaire 1500, 2 fonctions acrylate par molécule, EBECRYL^{®} 8402 : masse molaire 1000, 2 fonctions acrylate par molécule, EBECRYL^{®} 8804 : masse molaire 1300, 2 fonctions acrylate par molécule, EBECRYL^{®} 220 : masse molaire 1000, 6 fonctions acrylate par molécule, EBECRYL^{®} 2220 : masse molaire 1200, 6 fonctions acrylate par molécule, EBECRYL^{®} 1290 : masse molaire 1000, 6 fonctions acrylate par molécule, EBECRYL^{®} 800 : masse molaire 800, 6 fonctions acrylate par molécule).
   On peut également citer les polyuréthannes aliphatiques diacrylate hydrosolubles commercialisés sous les dénominations EBECRYL^{®} 2000, EBECRYL^{®} 2001 et EBECRYL^{®} 2002, et les polyuréthannes diacrylate en dispersion aqueuse commercialisés sous les dénominations commerciales IRR^{®} 390, IRR^{®} 400, IRR^{®} 422 IRR^{®} 424 par la société UCB.
d) les polyéthers à groupes (méth)acrylate obtenus par estérification, par l'acide (méth)acrylique, des groupes hydroxyle terminaux d'homopolymères ou de copolymères d'alkylèneglycols en C₁-C₄, tels que le polyéthylèneglycol, le polypropylèneglycol, les copolymères d'oxyde d'éthylène et d'oxyde de propylène ayant de préférence une masse moléculaire moyenne en poids inférieure à 10 000, le triméthylolpropane polyéthoxylé ou polypropoxylé.
   Des polyoxyéthylènes-di(méth)acrylate de masse molaire appropriée sont commercialisés par exemple sous les dénominations SR 259, SR 344, SR 610, SR 210, SR 603 et SR 252 par la société CRAY VALLEY ou sous la dénomination EBECRYL® 11 par UCB. Des triacrylates de triméthylolpropane polyéthoxylé sont commercialisés par exemple sous les dénominations SR 454, SR 498, SR 502, SR 9035, SR 415 par la société CRAY VALLEY ou sous la dénomination EBECRYL® 160 par la société UCB. Des triacrylates de triméthylolpropane polypropoxylé sont commercialisés par exemple sous les dénominations SR 492 et SR 501 par la société CRAY VALLEY.
e) les époxyacrylates obtenus par réaction entre :
   - au moins un diépoxyde choisi par exemple parmi :
      (i) l'éther diglycidylique de bisphénol A,
      (ii) une résine diépoxy résultant de la réaction entre l'éther diglycidylique de bisphénol A et l'épichlorhydrine,
      (iii) une résine époxyester à extrémités α,ω-diépoxy résultant de la condensation d'un diacide carboxylique ayant de 3 à 50 atomes de carbone avec un excès stoechiométrique de (i) et / ou (ii),
      (iv) une résine époxyéther à extrémités α,ω-diépoxy résultant de la condensation d'un diol ayant de 3 à 50 atomes de carbone avec un excès stoechiométrique de (i) et / ou (ii),
      (v) les huiles naturelles ou synthétiques portant au moins 2 groupes époxyde, telles que l'huile de soja époxydée, l'huile de lin époxydée et l'huile de vernonia époxydée,
      (vi) un polycondensat phénol-formaldéhyde (résine Novolac^{®}), dont les extrémités et/ou les groupes latéraux ont été époxydés,
      et
   - un ou plusieurs acides carboxyliques ou polyacides carboxyliques comportant au moins une double liaison éthylénique en α,β du groupe carboxylique comme l'acide (méth)acrylique ou l'acide crotonique ou les esters d'acide (méth)acrylique et d'un diol ou polyol ayant de 2 à 20 atomes de carbone, de préférence de 2 à 6 atomes de carbone tels que le (méth)acrylate de 2-hydroxyéthyle.
   De tels polymères sont commercialisés par exemple sous les dénominations SR 349, SR 601, CD 541, SR 602, SR 9036, SR 348, CD 540, SR 480, CD 9038 par le société CRAY VALLEY, sous les dénominations EBECRYL® 600 et EBECRYL® 609, EBECRYL® 150, EBECRYL® 860, EBECRYL® 3702 par la société UCB et sous les dénominations PHOTOMER^{®} 3005 et PHOTOMER^{®} 3082 par la société HENKEL.
f) les poly(méth)acrylates d'(alkyle en C₁₋₅₀), ledit alkyle étant linéaire, ramifié ou cyclique, comportant au moins deux fonctions à double liaison éthylénique portées par les chaînes hydrocarbonées latérales et/ou terminales.
   De tels copolymères sont commercialisés par exemple sous les dénominations IRR® 375, OTA^{®} 480 et EBECRYL^{®}2047 par la société UCB.
g) les polyoléfines telles que le polybutène, le polyisobutylène,
h) les perfluoropolyéthers à groupes acrylate obtenus par estérification, par exemple par l'acide (méth)acrylique, de perfluoropolyéthers portant des groupes hydroxyle latéraux et / ou terminaux.
   De tels perfluoropolyéthers α,ω-diots sont décrits notamment dans EP-A-1057849 et sont commercialisés par la société AUSIMONT sous la dénomination FOMBLIN^{®} Z DIOL.
i) les dendrimères et polymères hyperramifiés portant des groupes terminaux (méth)acrylate ou (méth)acrylamide obtenus respectivement par estérification ou amidification de dendrimères et de polymères hyperramifiés à fonctions terminales hydroxyle ou amino, par de l'acide (méth)acrylique.

Les dendrimères (du grec dendron = arbre) sont des molécules polymères "arborescentes", c'est-à-dire très ramifiées inventées par D. A. Tomalia et son équipe au début des années 90 (Donald A. Tomalia et al., Angewandte Chemie, Int. Engl. Ed., vol. 29, n° 2, pages 138 - 175). Il s'agit de structures construites autour d'un motif central généralement polyvalent. Autour de ce motif central, sont enchaînés, selon une structure parfaitement déterminée, des motifs ramifiés d'allongement de chaîne donnant ainsi naissance à des macromolécules symétriques, monodispersées ayant une structure chimique et stéréochimique bien définie. Des dendrimères de type polyamidoamine sont commercialisés par exemple sous la dénomination STARBUST^{®} par la société DENDRITECH.

Les polymères hyperramifiés sont des polycondensats, généralement de type polyester, polyamide ou polyéthylèneamine, obtenus à partir de monomères multifonctionnels, qui ont une structure arborescente similaire à celle des dendrimères mais beaucoup moins régulière que celle-ci (voir par exemple WO-A-93/17060 et WO 96/12754).

La société PERSTORP commercialise sous la dénomination BOLTORN^{®} des polyesters hyperramifiés. On trouvera sous la dénomination COMBURST^{®} de la société DENDRITECH des polyéthylèneamines hyperramifiées. Des poly(esteramide) hyperramifiés à extrémités hydroxyle sont commercialisés par la société DSM sous la dénomination HYBRANE^{®}.

Ces dendrimères et polymères hyperramifiés estérifiés ou amidifiés par l'acide acrylique et/ou méthacrylique se distinguent des polymères décrits sous les points a) à h) ci-dessus par le très grand nombre de doubles liaisons éthyléniques présentes. Cette fonctionnalité élevée, le plus souvent supérieure à 5, les rend particulièrement utiles en leur permettant de jouer un rôle de "noeud de réticulation", c'est-à-dire de site de réticulation multiple.

On peut donc utiliser ces polymères dendritiques et hyperramifiés en association avec un ou plusieurs des polymères et/ou oligomères a) à h) ci-dessus.

### 1a Composés réactifs additionnels

Selon un mode de réalisation, la composition conforme à l'invention peut comprendre en outre un composé réactif additionnel tel que :
- les particules organiques ou minérales comprenant à leur surface au moins 2 groupements aliphatiques insaturés, on peut citer par exemple les silices traitées en surface par exemple par des composés siliconés à groupements vinyliques tels que par exemple la silice traitée cyclotetramethyltetravinylsiloxane,
- des composés silazanes tels que l'hexaméthyldisilazane.

### 1b Catalyseur

La réaction d'hydrosilylation se fait avantageusement en présence d'un catalyseur qui peut être présent dans la composition conforme à l'invention, le catalyseur étant de préférence à base de platine ou d'étain.

On peut citer par exemple les catalyseurs à base de platine déposé sur un support de gel de silice ou de poudre de charcoal (charbon), le chlorure de platine, les sels de platine et d'acides chloroplatiniques.

On utilise de préférence les acides chloroplatiniques sous forme hexahydrate ou anhydre, facilement dispersible dans les milieux organosiliconés.

On peut également citer les complexes de platine tels que ceux à base d'acide chloroplatinique hexahydrate et de divinyl tétraméthyldisiloxane.

Le catalyseur peut être présent dans la composition conforme à la présente invention en une teneur allant 0,0001 % à 20 % en poids par rapport au poids total de la composition.

On peut également introduire dans la composition de l'invention des inhibiteurs ou retardateurs de polymérisation, et plus particulièrement des inhibiteurs du catalyseur, ceci afin d'accroître la stabilité de la composition dans le temps ou de retarder la polymérisation. De façon non limitative, on peut citer les polyméthylvinylsiloxanes cycliques, et en particulier le tétravinyl tétraméthyl cyclotétrasiloxane, les alcools acétyléniques, de préférence volatiles, tels que le méthylisobutynol.

La présence de sels ioniques, tels que l'acétate de sodium, dans la composition peut avoir une influence dans la vitesse de polymérisation des composés.

De façon avantageuse, les composés X et Y sont choisis parmi les composés siliconés susceptibles de réagir par hydrosilylation ; en particulier le composé X est choisi parmi les polyorganosiloxanes comprenant des unités de formule (1) décrits ci-dessus et le composé Y est choisi parmi les organosiloxanes comprenant des unités alkylhydrogènosiloxanes de formule (III) décrits ci-dessus.

Selon un mode de réalisation particulier, le composé X est un polydiméthylsiloxane à groupements vinyliques terminaux, et le composé Y est le méthylhydrogénosiloxane.

A titre d'exemple d'une combinaison de composés X et Y réagissant par hydrosilylation, on peut citer les références suivantes proposée par la société Dow Corning : DC 7-9800 Soft Skin Adhesive Parts A & B, ainsi que les mélanges A et B suivants préparés par Dow Corning:
MELANGE A :

| Ingrédient (Nom INCl) | N°CAS | Teneurs (%) | Fonction |
|---|---|---|---|
| Dimethyl Siloxane, Dimethylvinylsiloxy-terminated | 68083-19-2 | 55-95 | Polymère |
| Silica Silylate | 68909-20-6 | 10-40 | Charge |
| 1,3-Diethenyl-1,1,3,3-Tetramethyldisiloxane complexes | 68478-92-2 | Trace | Catalyseur |
| Tetramethyldivinyldisiloxane | 2627-95-4 | 0.1-1 | Polymère |

MELANGE B:

| Ingrédient (Nom INCl) | N°CAS | Teneurs (%) | Fonction |
|---|---|---|---|
| Dimethyl Siloxane, Dimethylvinylsiloxy-terminated | 68083-19-2 | 55-95 | Polymère |
| Silica Silylate | 68909-20-6 | 10-40 | Charge |
| Dimethyl, Methylhydrogen Siloxane, trimethylsiloxy-terminated | 68037-59-2 | 1-10 | Polymère |

### 2/ Composés X et Y susceptibles de réagir par condensation

Selon ce mode de réalisation, les composés X et Y sont susceptibles de réagir par condensation, soit en présence d'eau (hydrolyse) par réaction de 2 composés porteurs de groupements alcoxysilanes, soit par condensation dite « directe » par réaction d'un composé porteur de groupement(s) alcoxysilane(s) et d'un composé porteur de groupement(s) silanol(s) ou par réaction de 2 composés porteurs de groupement(s) silanol(s).

Lorsque la condensation se fait en présence d'eau, celle-ci peut être en particulier l'humidité ambiante, l'eau résiduelle des fibres kératiniques, ou l'eau apportée par une source extérieure, par exemple par humidification préalable des fibres kératiniques (par exemple par un brumisateur).

Dans ce mode de réaction par condensation, les composés X et Y, identiques ou différents, peuvent donc être choisis parmi les composés siliconés dont la chaîne principale comprend au moins deux groupes alcoxysilane et / ou au moins deux groupes silanol (Si-OH), latéraux et / ou en bout de chaîne.

Selon un mode de réalisation avantageux, les composés X et / ou Y sont choisis parmi les polyorganosiloxanes comprenant au moins deux groupes alcoxysilane. Par groupe « alcoxysilane », on entend un groupe comprenant au moins une partie -Si-OR, R étant un groupe alkyle comprenant de 1 à 6 atomes de carbone.

Les composés X et Y sont notamment choisis parmi les polyorganosiloxanes comprenant des groupes terminaux alcoxysilanes, plus spécifiquement ceux qui comprennent au moins 2 groupes alcoxysilanes terminaux, de préférence trialcoxysilanes terminaux.

Ces composés X et / ou Y comprennent de préférence de façon majoritaire des unités de formule : 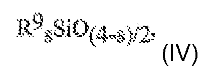 dans laquelle R⁹ représente indépendamment un radical choisi parmi les groupes alkyle comprenant de 1 à 6 atomes de carbone, le phényle, les groupes alkyle fluorés, et s est égal à 0, 1, 2 ou 3. De préférence, R⁹ représente indépendamment un groupe alkyle comprenant de 1 à 6 atomes de carbone. Comme groupe alkyle, on peut citer notamment le méthyle, le propyle, le butyle, l'hexyle et leurs mélanges, de préférence le méthyle ou l'éthyle. Comme groupe fluoroalkyle, on peut citer le 3, 3, 3-trifluoropropyle.

Selon un mode de réalisation particulier, les composés X et Y, identiques ou différents, sont des polyorganosiloxanes comprenant des unités de formule :

(R⁹₂SiO₂)_{f} - (V)

dans laquelle R⁹ est tel que décrit ci-dessus, de préférence R⁹ est un radical méthyle, et
f est notamment tel que le polymère présente une viscosité à 25 °C allant de 0,5 à 3000 Pa.s, de préférence allant de 5 à 150 Pa.s et / ou
f est notamment un nombre allant de 2 à 5000, de préférence de 3 à 3000, mieux encore de 5 à 1000.

Ces composés X et Y polyorganosiloxanes comprennent au moins 2 groupes trialcoxysilanes terminaux par molécule de polymère, lesdits groupes ayant la formule suivante :

ZSiR¹ₓ(OR)₃₋ₓ, (VI)

dans laquelle :
les radicaux R représentent indépendamment un groupe méthyle, éthyle, n-propyle, isopropyle, n-butyle, sec-butyle, isobutyle, de préférence un groupe méthyle ou éthyle,
R¹ est un groupe méthyle ou éthyle,
x est égal à 0 ou 1, de préférence x est égal à 0, et
Z est choisi parmi : les groupes hydrocarbonés divalents ne comportant pas d'insaturation éthylénique et comprenant de 1 à 18 atomes de carbone (groupes alkylène), les combinaisons de radicaux hydrocarbonés divalents et de segments siloxanes de formule (IX) suivante : 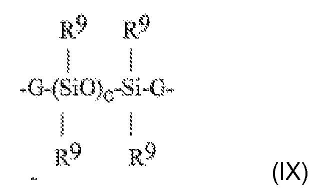
R⁹ étant tel que décrit plus haut, G est un radical hydrocarboné divalent ne comportant pas d'insaturation éthylénique et comprenant de 1 à 18 atomes de carbone et c est un entier allant de 1 à 6.

Z et G peuvent être notamment choisis parmi les groupements alkylène tels que le méthylène, l'éthylène, le propylène, le butylène, le pentylène, l'hexylène, les groupements arylène tels que le phénylène.

De préférence, Z est un groupe alkylène, et mieux éthylène.

Ces polymères peuvent présenter en moyenne au moins 1,2 groupements terminaux ou chaînes terminales trialcoxysilanes par molécule, et de préférence en moyenne au moins 1,5 groupements terminaux trialcoxysilanes par molécule. Ces polymères pouvant présenter au moins 1,2 groupements terminaux trialcoxysilanes par molécule, certains peuvent comprendre d'autre types de groupes terminaux tels que des groupements terminaux de formule CH₂=CH-SiR⁹₂- ou de formule R⁶₃- Si-, dans laquelle R⁹ est tel que défini plus haut et chaque groupe R⁶ est indépendamment choisi parmi les groupes R⁹ ou vinyle. On peut citer comme exemples de tels groupements terminaux les groupes triméthoxysilane, triéthoxysilane, vinyldiméthoxysilane et vinylméthyloxyphénylsilane.

De tels polymères sont notamment décrits dans les documents US 3 175 993, US 4 772 675, US 4 871 827, US 4 888 380, US 4 898 910, US 4 906 719 et US 4 962 174 dont le contenu est incorporé par référence à la présente demande.

On peut citer à titre de composé X et / ou Y en particulier le polymère de formule : 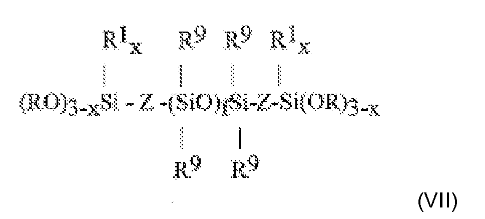 dans laquelle R, R¹, R⁹, Z, x et f sont tels que décrits plus haut.

Les composés X et / ou Y peuvent également comprendre un mélange de polymère de formule (VII) ci-dessus avec des polymères de formule (VIII) suivante : 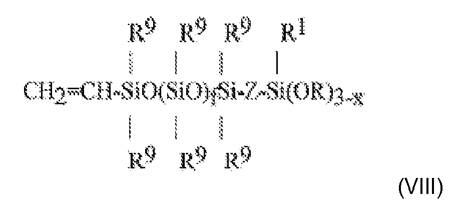 dans laquelle R, R¹, R⁹, Z, x et f sont tels que décrits plus haut.

Lorsque le composé X et / ou Y polyorganosiloxanes à groupe(s) alcoxysilane(s) comprend un tel mélange, les différents polyorganosiloxanes sont présents en des teneurs telles que les chaînes organosilyle terminales représentent moins de 40 %, de préférence moins de 25 % en nombre des chaînes terminales.

Les composés X et / ou Y polyorganosiloxanes particulièrement préférés sont ceux de formule (VII) décrits ci-dessus. De tels composés X et / ou Y sont décrits par exemple dans le document WO 01/96450.

Comme indiqué plus haut, les composés X et Y peuvent être identiques ou différents.

Selon une variante, l'un des 2 composés réactifs X ou Y est de nature silicone et l'autre est de nature organique. Par exemple le composé X est choisi parmi les oligomères ou polymères organiques ou les oligomères ou polymères hybrides organique / silicone, lesdits polymères ou oligomères comprenant au moins deux groupements alcoxysilanes et Y est choisi parmi les composés siliconés tels que les polyorganosiloxanes décrits ci-dessus. En particulier, les oligomères ou polymères organiques sont choisis parmi les oligomères ou polymères vinyliques, (méth)acryliques, polyesters, polyamides, polyuréthanes et / ou polyurées, polyéthers, polyoléfines, perfluoropolyéthers, dendrimères et polymères hyper-ramifiés organiques, et leurs mélanges.

Les polymères organiques de nature vinylique ou (méth)acrylique, porteurs de groupes latéraux alcoxysilanes, pourront en particulier être obtenus par copolymérisation d'au moins un monomère organique vinylique ou (méth)acrylique avec un (méth)acryloxypropyltriméthoxysilane, un vinyl triméthoxysilane, un vinyltriéthoxysilane, un allyltriméthoxysilanes, etc.

On peut citer par exemple les polymères (méth)acryliques décrits dans le document de KUSABE.M, Pitture e Verniei - European Coating ; 12-B, pages 43-49, 2005, et notamment les polyacrylates à groupes alcoxysilanes référencés MAX de Kaneka ou ceux décrits dans la publication de PROBSTER, M, Adhesion-Kleben & Dichten, 2004, 481 (1-2), pages 12-14.

Les polymères organiques résultant d'une polycondensation ou d'une polyaddition, tel que les polyesters, polyamides, polyuréthanes et / ou polyurées, polyéthers, et porteurs de groupes alcoysilanes latéraux et / ou terminaux, pourront résulter par exemple de la réaction d'un prépolymère oligomère tel que décrit plus haut avec l'un des co-réactifs silanes suivant porteurs d'au moins un groupe alcoxysilane : aminopropyltriméthoxysilane, aminopropyltriéthoxysilane, aminoéthylaminopropyltriméthoxysilane, glycidoxypropyltriméthoxysilane, glycidoxypropyltriéthoxysilane, époxycyclohéxyléthyltriméthoxysilane, mercaptopropyltriméthoxysilane.

Des exemples de polyéthers et de polyisobutylènes à groupes alcoxysilanes sont décrits dans la publication de KUSABE.M., Pitture e Verniei - European Coating ; 12-B, pages 43-49, 2005. Comme exemple de polyuréthanes à groupes alcoxysilanes terminaux , on peut citer ceux décrits dans le document PROBSTER, M., Adhesion-Kleben & Dichten, 2004, 481 (1-2), pages 12-14 ou encore ceux décrits dans le document LANDON, S., Pitture e Verniei vol. 73, N° 11 , pages 18-24, 1997 ou dans le document HUANG, Mowo, Pitture e Verniei vol. 5, 2000, pages 61-67, on peut notamment citer les polyuréthanes à groupes alcoxysilanes de OSI-WITCO-GE.

A titre de composés X et / ou Y polyorganosiloxanes, on peut citer les résines de type MO ou MT portant elle-même des extrémités alcoxysilanes et / ou silanols comme par exemple les résines poly(isobutylsilsesquioxane) fonctionnalisées par des groupes silanols proposées sous la référence SST-S7C41 (3 groupes Si-OH) par la société Gelest.

### 2a Composés réactifs additionnels

La composition conforme à la présente invention peut comprendre en outre un composé réactif additionnel comprenant au moins deux groupes alcoxysilane ou silanol.

On peut citer par exemple une ou des particules organiques ou minérales comprenant à leur surface des groupement alcoxysilanes et / ou silanols, par exemple des charges traitées en surface par de tels groupes.

### 2b Catalyseur

La réaction de condensation peut se faire en présence d'un catalyseur à base de métal qui peut être présent dans la composition conforme à l'invention. Le catalyseur utile dans ce type de réaction est de préférence un catalyseur à base de titane.

On peut citer notamment les catalyseurs à base de tétraalcoxytitane de formule :

Ti(OR²)_{y}(OR³)_{4-y},

dans laquelle R² est choisi parmi les radicaux alkyle tertiaires tels que le tert butyle, le tert amyle et le 2,4-diméthyl-3-pentyle ; R³ représente un radical alkyle comprenant de 1 à 6 atomes de carbone, de préférence un groupe méthyle éthyle, n-propyle, isopropyle, n-butyle, sec-butyle, hexyle et y est un nombre allant de 3 à 4, mieux de 3,4 à 4.

Le catalyseur peut être présent dans la composition conforme à la présente invention en une teneur allant 0,0001 % à 20 % en poids par rapport au poids total de la composition.

### 2c Diluant

La composition conforme à l'invention peut comprendre en outre une huile siliconée volatile (ou diluant) destinée à faire diminuer la viscosité de la composition. Cette huile peut être choisie parmi les silicones linéaires à chaîne courte telles que l'hexaméthyldisiloxane, l'octaméthyltrisiloxane, les silicones cycliques telles que l'octaméthylcyclotétrasiloxane, la décaméthylclopentasiloxane et leurs mélanges.

Cette huile siliconée peut représenter de 5 % à 95 %, de préférence de 10 à 80 % en poids par rapport au poids de chaque composition.

A titre d'exemple d'une combinaison de composés X et Y porteurs de groupements alcoxysilanes et réagissant par condensation, on peut citer la combinaison des mélanges A' et B' suivants préparés par la société Dow Corning :
**Mélange A' :**

| **Ingrédient (Nom INCI)** | **N°CAS** | **Teneurs (%)** | **Fonction** |
|---|---|---|---|
| Bis-Trimethoxysiloxyethyl Tetramethyldisiloxyethyl Dimethicone (1) | PMN87176 | 25-45 | Polymère |
| Silica Silylate | 68909-20-6 | 5-20 | Charge |
| Disiloxane | 107-46-0 | 30-70 | Solvant |

**Mélange B' :**

| **Ingrédient (Nom INCl)** | **N°CAS** | **Teneurs (%)** | **Fonction** |
|---|---|---|---|
| Disiloxane | 107-46-0 | 80-99 | Solvant |
| Tetra T Butyl Titanate | - | 1-20 | Catalyseur |

Il est d'ailleurs à noter que les composés X et Y identiques sont réunis dans le mélange A'.

### 3/ Réticulation en présence de peroxyde :

Cette réaction se fait de préférence par chauffage à une température supérieure ou égale à 50 °C, de préférence supérieure ou égale à 80 °C, allant jusqu'à 120 °C.

Les composés X et Y, identiques ou différents, comprennent dans ce cas au moins deux groupements latéraux -CH₃ et / ou au moins deux chaînes latérales portant un groupement -CH₃.

Les composés X et Y sont de préférence siliconés et peuvent être choisis par exemple parmi les polydiméthylsiloxanes linéaires non volatiles de haut poids moléculaire, ayant un degré de polymérisation supérieur à 6 présentant au moins deux groupements latéraux -CH₃ reliés à l'atome de silicium et / ou au moins deux chaînes latérales portant un groupement -CH₃. On peut citer par exemple les polymères décrits dans le Catalogue « Reactive Silicones » de la société Gelest Inc., Edition 2004, page 6, et notamment les copolymères (aussi appelés gommes) de vinylméthylsiloxane-diméthylsiloxane de poids moléculaires allant de 500 000 à 900 000 et de viscosité supérieure à 2 000 000 cSt.

A titre de peroxydes utilisables dans le cadre de l'invention, on peut citer le peroxyde de benzoyle, le peroxyde de 2,4-dichlorobenzoyle et leurs mélanges.

Selon un mode de réalisation, la réaction d'hydrosilylation ou la réaction de condensation ou bien encore la réaction de réticulation en présence d'un peroxyde entre les composés X et Y est accélérée par un apport de chaleur en élevant par exemple la température du système entre 25 °C et 180 °C. Le système réagira notamment sur les fibres kératiniques.

D'une façon générale, quel que soit le type de réaction par laquelle les composés X et Y réagissent ensemble, le pourcentage molaire de X par rapport à l'ensemble des composés X et Y, c'est-à-dire le ratio X / (X+Y) x 100, peut varier de 5 % à 95 %, de préférence de 10 % à 90 %, mieux encore de 20 % à 80 %.

De même, le pourcentage molaire de Y par rapport à l'ensemble des composés X et Y, c'est-à-dire le ratio Y / (X+Y) x 100, peut varier de 5 % à 95 %, de préférence de 10 % à 90 %, mieux encore de 20 % à 80 %.

Le composé X peut présenter une masse moléculaire moyenne en poids (Mw) allant de 150 à 1 000 000, de préférence de 200 à 800 000, de préférence encore de 200 à 250 000.

Le composé Y peut présenter une masse moléculaire moyenne en poids (Mw) allant de 200 à 1 000 000, de préférence de 300 à 800 000, de préférence encore de 500 à 250 000.

Le composé X peut représenter de 0,5 % à 95 % en poids par rapport au poids total de la composition, de préférence de 1 % à 90 % et mieux de 5 % à 80 %.

Le composé Y peut représenter de 0,05 % à 95 % en poids par rapport au poids total de la composition, de préférence de 0,1 % à 90 % et mieux de 0,2 % à 80 %.

Le ratio entre les composés X et Y peut être varié de manière à moduler la vitesse de réaction et donc la vitesse de formation du film ou encore de manière à adapter les propriétés du film formé (par exemple ses propriétés adhésives) selon l'application recherchée.

En particulier, les composés X et Y peuvent être présents en un ratio X / Y molaire allant de 0,05 à 20 et mieux de 0,1 à 10.

### Les agents texturisants

La composition appliquée sur les fibres capillaires peut en outre comprendre un ou plusieurs agents texturisants (charges), différents des pigments présents dans la composition.

On entend par agents texturisants, des particules minérales ou de synthèse, lamellaires ou non lamellaires, insolubles dans l'eau.

A titre d'exemple, ces agents texturisants peuvent être du carbonate de calcium colloïdal qui peut être traité ou non par de l'acide stéarique ou du stéarate, de la silice telle que les silices pyrogénées les silices précipitées, les silices traitées pour les rendre hydrophobes, du quartz moulu, de l'alumine, de l'hydroxyde d'aluminium, de la terre de diatomée, du dioxyde de titane, de la terre de diatomée, de l'oxyde de fer, du noir de carbone.

On peut aussi citer le talc, le mica, le kaolin, les poudres de polyamide (Nylon@) (Orgasol de chez Atochem), les poudres de polyéthylène, les poudres de polymères de tétrafluoroéthylène (TéflonB), l'amidon, des microsphères polymériques telles que celles de chlorure de polyvinylidène/acrylonitrile comme l'Expancel (Nobel Industrie), de copolymères d'acide acrylique (PolytrapB de la société Dow Corning).

Les silices synthétiques dont la surface est modifiée par des composés siliconés pour les rendre hydrophobe en surface sont particulièrement préférées. Ces charges se différentient les unes des autres par leurs propriétés de surface, les composés de silicone utilisés pour traiter la silice, et la façon dont le traitement en surface est réalisé.

De tels agents permettent de modifier la viscosité de la formulation obtenue à partir des composés X et/ou Y et/ou de modifier les propriétés du matériau obtenu.

On préfère à titre d'agent texturisant la silice, le carbonate de calcium, les agents à base de résine.

A titre d'exemple, on peut citer les silices traitées Cab-O-Sil@TS-530, Aerosil@R8200 et Wacker HDX H2000.

Les agents texturisants peuvent être présents à raison de 0 à 48 % en poids, par rapport au poids total de la composition, de préférence 0,01 à 30 % en poids, et mieux de 0,02 % à 20 % en poids.

Lorsque les composés X et Y sont susceptibles de réagir ensemble par une réaction de réticulation, on applique sur les fibres kératiniques au moins un peroxyde tel que défini précédemment.

Le ou les peroxydes peuvent être présents dans l'une ou l'autre ou dans plusieurs des compositions appliquées sur les fibres kératiniques déjà citées ou dans une composition supplémentaire, auquel cas l'ordre d'application des différentes compositions sur les fibres kératiniques est indifférent.

Selon un mode de réalisation particulier de l'invention, on applique sur les fibres kératiniques au moins un catalyseur tel que défini précédemment pour activer la réaction entre le ou les composés X et le ou les composés Y.

Par exemple, le ou les catalyseurs peuvent être présents dans l'une ou l'autre ou dans plusieurs des compositions appliquées sur les fibres kératiniques déjà citées ou dans une composition supplémentaire, auquel cas l'ordre d'application des différentes compositions sur les fibres kératiniques est indifférent.

Les catalyseurs avantageusement choisis sont ceux qui sont décrits ci-dessus.

Lorsque l'on applique sur les fibres kératiniques au moins un catalyseur et / ou au moins un peroxyde, le ou les composés X, le ou les composés Y, le ou les catalyseurs et / ou le ou les peroxydes ne sont pas stockés simultanément dans la même composition. Ils peuvent être en revanche mélangés au moment de l'emploi.

Selon un autre mode de réalisation particulier de l'invention, on applique sur les fibres kératiniques au moins un composé réactif additionnel.

Par exemple, le ou les composés réactifs additionnels peuvent être présents dans l'une ou l'autre ou dans plusieurs des compositions appliquées sur les fibres kératiniques déjà citées ou dans une composition supplémentaire, auquel cas l'ordre d'application des différentes compositions sur les fibres kératiniques est indifférent.

Les différentes compositions mises en oeuvre dans le procédé conforme à l'invention peuvent être appliquées sur des cheveux secs ou humides.

Un séchage intermédiaire et/ou un rinçage peut être réalisé entre chaque application.

L'utilisation du fer peut avoir lieu entre les applications des compositions séparées de X et Y, contenant d'une part le composé X et d'autre part le composé Y. De préférence, on utilise le fer après l'application de X et de Y.

Chaque composition utile dans le procédé conforme à l'invention peut contenir en outre divers additifs cosmétiques conventionnels.

Chaque composition utile dans le procédé conforme à l'invention comprend un milieu cosmétiquement acceptable, véhiculant le ou les composés X et / ou le ou les composés Y, et choisi de telle sorte que les composés X et Y soient aptes à réagir l'un avec l'autre par réaction d'hydrosilylation, de condensation, ou de réticulation en présence de peroxyde après l'application de la composition cosmétique sur les cheveux.

Le dépôt ainsi formé présente l'avantage d'avoir une faible solubilité attendue. En outre, il possède une bonne affinité pour la surface des fibres kératiniques, ce qui garantit une meilleure rémanence de l'ensemble du dépôt.

Lorsque les composés X et Y sont appliqués séparément, le dépôt en couches obtenu peut aussi être avantageux pour conserver les propriétés cosmétiques ou optiques du composé qui constitue la partie supérieure du dépôt.

Selon les mêmes procédés, il est possible de réaliser des superpositions multiples de couches de composés X et Y en alternance ou non pour atteindre le type de dépôt souhaité (en termes de nature chimique, résistance mécanique, épaisseur, aspect, toucher...).

### Solvants organiques

Par solvant organique, on entend une substance organique liquide à la température de 25 °C et à la pression atmosphérique (760 mm de Hg) capable de dissoudre une autre substance sans la modifier chimiquement.

Le ou les solvants organiques utiles dans le cadre de l'invention sont distincts des composés X et Y utiles dans le cadre de l'invention.

Le ou les solvants organiques sont par exemple choisis parmi les alcools aromatiques tels que l'alcool benzylique, le phénoxyéthanol, l'alcool phényléthylique ; les alcools gras liquides, notamment en C₁₀-C₃₀ les alcanols en C₁-C₆ tels que l'éthanol, l'isopropanol, le n-propanol, le butanol, le n-pentanol, le 1,2-propanediol, le 1,3-propanediol, le 1-méthoxy 2-propanol, le 1-éthoxy 2-propanediol, les 1,3 et 1,4-butanediol, le 1,2-hexanediol ; les polyols et éthers de polyols possédant une fonction -OH libre tels que le 2-butoxyéthanol, le propylèneglycol, le monométhyléther de propylèneglycol, le monoéthyléther et le monométhyléther du diéthylèneglycol, l'éthylène glycol monoéthyléther, l'éthylène glycol mono butyl éther, le néopentyl glycol, l'isoprèneglycol, le glycérol, le glycol, le dipropylène glycol, le butylène glycol, le butyle diglycol ; les silicones volatiles telles que les silicones linéaires à chaîne courte telle que l'hexaméthyldisiloxane, les silicones cycliques telles que l'octaméthylcyclotétradisiloxane, la décaméthylcyclopentasiloxane et la dodécaméthylcyclohexasiloxane, les polydiméthylsiloxanes modifiées ou non par des fonctions alkyle et / ou amine et / ou imine et / ou fluoroalkyl et / ou carboxylique et / ou betaïne et / ou ammonium quaternaire ; les polydiméthylsiloxanes modifiées liquides ; les huiles minérales, organiques ou végétales ; les alcanes et plus particulièrement les alcanes de C₅ à C₂₀ ; les acides gras liquides ; les esters gras liquides et plus particulièrement les benzoates ou les salicylates d'alcool gras liquides.

Le ou les solvants organiques sont de préférence choisis parmi les huiles organiques ; les silicones telles que les silicones volatiles, les gommes ou huiles de silicones aminés ou non et leurs mélanges ; les huiles minérales ; les huiles végétales telles que les huiles d'olive, de ricin, de colza, de coprah, de germe de blé, d'amande douce, d'avocat, de macadamia, d'abricot, de carthame, de noix de bancoulier, de camélina, de tamanu, de citron ou encore des composés organiques tels que des alcanes en C₅-C₂₀, l'acétone, la méthyléthylcétone, les esters d'acides en C₁-C₂₀ liquides et d'alcools en C₁-C₈ tels que l'acétate de méthyle, l'acétate de butyle, l'acétate d'éthyle et le myristate d'isopropyle, le diméthoxyéthane, le diéthoxyéthane, les alcools gras liquides en C₁₀-C₃₀ tels que l'alcool oléique, les esters d'alcools gras ou d'alcool gras liquides tels que les benzoates d'alcool gras en C₁₀-C₃₀ et leurs mélanges ; l'isononanoate d'isononyle, le malate d'isostéaryle, le tétra-isostéarate de pentaérythrityle, le trimélate de tridécyle ; l'huile de polybutène ; le mélange cyclopentasiloxane (14,7 % en poids) / polydiméthylsiloxane dihydroxylé en positions α et w (85,3 % en poids), ou leurs mélanges.

Selon un mode de réalisation préféré, le ou les solvants organiques sont choisis parmi les silicones telles que les polydiméthylsiloxanes liquides et les polydiméthylsiloxanes modifiées liquides, leur viscosité à 25 °C étant comprise entre 0,1 est et 1 000 000 cst, et plus préférentiellement entre 1 cst et 30 000 cst.

On citera de préférence les huiles suivantes :
- le mélange de polydiméthylsiloxane alpha-omega-dihydroxylé / cyclopentadiméthylsiloxane (14,7 / 85,3) commercialisé par Dow Corning sous le nom de DC 1501 Fluid ;
- le mélange de polydiméthylsiloxane alpha-omega-dihydroxylé / polydiméthylsiloxane commercialisé par Dow Corning sous le nom de DC 1503 Fluid ;
- le mélange de diméthicone / cyclopentadiméthylsiloxane commercialisé par Dow Corning sous le nom de DC 1411 Fluid ou celui commercialisé par Bayer sous le nom SF1214 ;
- la cyclopentadiméthylsiloxane commercialisée par Dow Corning sous le nom de DC245 Fluid ;
et les mélanges respectifs de ces huiles.

Ces solvants organiques peuvent servir de diluant pour les réactions de polycondensation.

Le ou les solvants organiques de la composition représentent généralement de 0,01 à 99 %, de préférence de 50 à 99 % en poids par rapport au poids total de la composition.

La composition de l'invention peut contenir, outre le ou les solvants organiques, de l'eau dans une proportion allant de 4 à 99% et de préférence de 1 à 50 % par rapport au poids total de la composition. La composition de l'invention peut aussi être anhydre, c'est-à-dire contenant moins de 1 % en poids d'eau par rapport au poids total de la composition.

La composition de l'invention peut aussi se présenter sous forme d'une émulsion et/ou être encapsulée. Lorsque la composition est une émulsion elle est par exemple constituée par une phase dispersée ou continue qui peut être de l'eau, des alcools aliphatiques en C₁-C₄ ou leurs mélanges, et une phase organique insoluble dans l'eau.

La composition conforme à l'invention peut également contenir, outre les composés X et Y de l'invention, au moins un agent utilisé habituellement en cosmétique choisi, par exemple, parmi des agents réducteurs, des corps gras, des plastifiants, des adoucissants, des agents anti-mousse, des agents hydratants, des pigments, des argiles, des charges minérales, des filtres UV, des colloïdes minéraux, des peptisants, des parfums, des conservateurs, des tensio-actifs anioniques, cationiques, non ioniques ou amphotères, des polymères fixants ou non, des protéines, des vitamines, des colorants directs, des colorants d'oxydation, des agents nacrants ou opacifiants, des propulseurs, et des épaississants minéraux ou organiques tels que le benzylidène-sorbitol et les N-acylaminoacides, les cires oxyéthylénées ou non, les paraffines, les acides gras solides en C₁₀-C₃₀ tels que l'acide stéarique, l'acide laurique, les amides gras en C₁₀-C₃₀ tels que le diéthanolamide laurique, les esters d'alcool gras ou d'acides gras solides, des azurants optiques.

Cette composition peut se présenter sous des formes diverses, telles que des lotions, des sprays, des mousses et être appliquées sous forme de shampooing ou d'après-shampooing.

Dans le cas des sprays, la composition de l'invention peut contenir un propulseur. Le propulseur est constitué par les gaz comprimés ou liquéfiés usuellement employés pour la préparation de compositions aérosols. On emploiera de manière préférentielle l'air, le gaz carbonique, l'azote comprimé ou encore un gaz soluble tel que le diméthyléther, les hydrocarbures halogénés (fluorés en particuliers) ou non et leurs mélanges.

On pourra aussi le cas échéant utiliser des aérosols à poche(s) contenant une ou plusieurs poches.

De préférence, les composés X et Y sont appliqués sur des fibres capillaires humides.

Avantageusement, après l'application de la ou des composition(s) comprenant X et Y, on laisse poser ladite composition pendant un temps compris entre 30 secondes et 60 minutes.

Le pH de la ou des composition(s) est généralement compris entre 2 et 13, de préférence entre 4 et 10.

Le réglage du pH de la composition peut être obtenu à l'aide d'un agent alcalin, tel que, par exemple, l'ammoniaque, la monoéthanolamine, la diéthanolamine, la triéthanolamine, la 1,3-propanediamine, un carbonate ou bicarbonate alcalin ou d'ammonium, un carbonate organique tel que le carbonate de guanidine, un hydroxyde alcalin, ou bien à l'aide d'un agent acidifiant tel que par exemple l'acide chlorhydrique, l'acide acétique, l'acide lactique, l'acide oxalique ou l'acide borique.

Généralement, la composition appliquée sur les fibres capillaires est appliquée à hauteur de 0,05 à 0,3 g, de préférence de 0,1 à 0,2 g de composition par gramme de fibre capillaire sèche.

Au sens de la présente invention, on entend par fer un dispositif de chauffage des fibres capillaires mettant en contact lesdites fibres et le dispositif de chauffage.

L'extrémité du fer venant en contact avec les cheveux présente deux surfaces qui peuvent être de différentes formes. Elles peuvent être planes (fer plat) ou arrondies (fer rond). Ces deux surfaces planes peuvent être métalliques. Elles peuvent être lisses ou crantées.

A titre d'exemple de fers utilisables dans le procédé selon l'invention, on peut citer tous types de fer et, en particulier, de manière non limitative, ceux décrits dans les brevets US 4 103 145, US 4 308 878, US 5 983 903, US 5 957 140, US 5 494 058 et US5046516.

L'application du fer peut se faire par touches séparées successives de quelques secondes, ou par déplacement ou glissement progressif le long des mèches.

De préférence, l'application du fer dans le procédé selon l'invention se fait en mouvement continu de la racine à la pointe, en un ou plusieurs passages.

Le procédé selon l'invention peut également comprendre une étape supplémentaire de pré-séchage partiel des fibres capillaires avant l'étape d'élévation de la température, de manière à éviter d'importants dégagements de vapeurs qui pourraient brûler les mains du coiffeur et le cuir chevelu du sujet. Cette étape de pré-séchage peut se faire par exemple au moyen d'un séchoir, d'un casque, ou bien encore par séchage libre.

Comme expliqué précédemment, le procédé selon l'invention permet d'améliorer le niveau de lissage des cheveux et de prolonger cet effet dans le temps.

La présente invention a donc également pour objet l'utilisation du procédé tel que décrit précédemment pour augmenter le lissage des fibres capillaires et prolonger la durabilité du lissage.

L'invention est illustrée par les exemples suivants.

### EXEMPLES

### Exemple 1 : réaction de condensation

Les compositions 1 et 2 telles que définies ci-dessous sont préparées. Dans ces exemples de composition, des mélanges A' et B' suivants préparés par la société Dow Corning :
**Mélange A' :**

| **Ingrédient (Nom INCl)** | **N°CAS** | **Teneurs (%)** | **Fonction** |
|---|---|---|---|
| Bis-Trimethoxysiloxyethyl Tetramethyldisiloxyethyl Dimethicone (1) | PMN87176 | 25-45 | Polymère |
| Silica Silylate | 68909-20-6 | 5-20 | Charge |
| Disiloxane | 107-46-0 | 30-70 | Solvant |

**Mélange B' :**

| | | | |
|---|---|---|---|
| **Ingrédient (Nom INCI)** | **N°CAS** | **Teneurs (%)** | **Fonction** |
| Disiloxane | 107-46-0 | 80-99 | Solvant |
| Tetra T Butyl Titanate | - | 1-20 | Catalyseur |

Il est d'ailleurs à noter que les composés X et Y identiques sont réunis dans le mélange A'.

### Exemple 2 Réaction d'hydrosilylation

Les compositions 3 et 4 telles que définies ci-dessous sont préparées. Dans ces exemples de composition, on utilise la combinaison des mélanges A1 et B1 suivants préparés par la société Dow Corning :
MELANGE A1 :

| Ingrédient (Nom INCl) | N°CAS | Teneurs (%) | Fonction |
|---|---|---|---|
| Dimethyl Siloxane, | 68083-19-2 | 55-95 | Polymère |
| Dimethylvinylsiloxy-terminated | | | |
| Silica Silylate | 68909-20-6 | 10-40 | Charge |
| 1,3-Diethenyl-1,1,3,3-Tetramethyldisiloxane complexes | 68478-92-2 | Trace | Catalyseur |
| Tetramethyldivinyldisiloxane | 2627-95-4 | 0.1-1 | Polymère |

MELANGE B1 :

| Ingrédient (Nom INCl) | N°CAS | Teneurs (%) | Fonction |
|---|---|---|---|
| Dimethyl Siloxane, Dimethylvinylsiloxyterminated | 68083-19-2 | 55-95 | Polymère |
| Silica Silylate | 68909-20-6 | 10-40 | Charge |
| Dimethyl, Methylhydrogen Siloxane, trimethylsiloxyterminated | 68037-59-2 | 1-10 | Polymère |

Pour les exemples 1 et 2, on met en oeuvre le procédé de traitement des fibres capillaires selon l'invention.

Les cheveux sont traités selon le procédé suivant :
- application de la composition formée par A1+B1, pour l'exemple 1 et A2+B2 pour l'exemple 2, sur les cheveux humides, la composition représentant environ 0,15 g par gramme de cheveux secs ;
- pré-séchage des cheveux au sèche-cheveux jusqu'à ce que les cheveux soient presque secs (on parle de pré-séchage à 80%) ;
- passage d'un fer (fer commercialisé sous la référence Techniliss loni Ceramic # PNC 228 par la société VELECTA), en un mouvement continu de la racine des cheveux à la pointe, en un ou plusieurs passages ; la température du fer est d'environ 210°C.

On obtient une mèche lisse, dont les cheveux sont individualisés.

On peut remplacer la composition formée par A1+B1, pour l'exemple 1 par la composition suivante, exprimé en pourcentage en poids :
A1+B1 ........................................................................ 37%
Silicone D5 commercialisée par Dow Corning.........10 %
Styleze W20 commercialisée par ISP......................0,5 %
Genamin KDMP commercialisé par Clariant ............0,5 %
Alcool cétylstéarylique................................................. 2 %
Eau déminéralisée........................................................qsp

On peut remplacer la composition formée par A2+B2, pour l'exemple 2 par la composition suivante, exprimée en pourcentage en poids :
A2+B2.......................................................................2 %
Silicone D5 commercialisée par Dow Corning......98 %

Après application du même protocole que pour les exemples 1 et 2, on obtient un très beau lissage avec une très bonne tenue des effets dans le temps, sans reprise de frisotis.

## Revendications

1. Procédé de traitement des fibres capillaires comprenant les étapes suivantes :
- application sur les fibres kératiniques d'au moins un composé X et au moins un composé Y, l'un au moins des composés X et Y étant un composé siliconé, lesdits composés X et Y étant susceptibles de réagir ensemble par une réaction d'hydrosilylation, de condensation, ou de réticulation en présence de peroxyde lorsqu'ils sont mis en contact l'un avec l'autre ;
- élévation de la température des fibres kératiniques, au moyen d'un fer chauffant, à une température au moins égale à 60°C, l'élévation de la température étant effectuée avant ou après un rinçage éventuel des fibres kératiniques ;
le ou les composés X, le ou les composés Y, étant appliqués sur les fibres kératiniques à partir de plusieurs compositions contenant le ou les composés X, le ou les composés Y, seuls ou en mélange, ou à partir d'une seule composition contenant le ou les composés X, le ou les composés Y.

2. Procédé selon la revendication 1 dans lequel on met en oeuvre un catalyseur pour faire réagir X et Y.

3. Procédé selon la revendication 1 ou 2 dans lequel les composés X et Y sont susceptibles de réagir par hydrosilylation.

4. Procédé selon la revendication 3 dans lequel le composé X est choisi parmi les composés siliconés comprenant au moins deux groupements aliphatiques insaturés.

5. Procédé selon la revendication 4 dans lequel le composé X est un polyorganosiloxane comprenant une chaîne principale siliconée dont les groupements aliphatiques insaturés sont pendants à la chaîne principale (groupe latéral).

6. Procédé selon la revendication 4 dans lequel le composé X est un polyorganosiloxane comprenant une chaîne principale siliconée dont les groupements aliphatiques insaturés sont situés aux extrémités de la chaîne principale du composé (groupe terminal).

7. Procédé selon l'une quelconque des revendication 3 à 6 dans lequel le composé X est choisi parmi les polyorganosiloxanes comprenant au moins deux groupements aliphatiques insaturés lié à un atome de silicium.

8. Procédé selon l'une quelconque des revendications 3 à 7 dans lequel le composé X est choisi parmi les polyorganosiloxanes comprenant des unités siloxanes de formule : 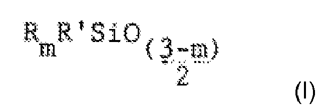 dans lequel :
- R représente un groupe hydrocarboné monovalent, linéaire ou cyclique, comprenant de 1 à 30 atomes de carbone;
- m est égal à 1 ou 2 ; et
- R' représente un groupement hydrocarboné aliphatique insaturé comprenant de 2 à 10 atomes de carbone ou un groupement hydrocarboné cyclique insaturé comprenant de 5 à 8 atomes de carbone.

9. Procédé selon la revendication 8 dans lequel le polyorganosiloxane de formule (I) est tel que R' représente un groupe vinyle ou un groupe -R"-CH=CHR"' dans lequel R" est une chaîne hydrocarbonée aliphatique divalente, comprenant de 1 à 8 atomes de carbone, liée à l'atome de silicium et R"' est un atome d'hydrogène ou un radical alkyle comprenant de 1 à 4 atomes de carbone.

10. Procédé selon la revendication 8 ou 9 dans lequel R représente un radical alkyle comprenant de 1 à 10 atomes de carbone ou encore un groupement phényle et R' est choisi parmi les groupements vinyle.

11. Procédé selon la revendication 10 dans lequel les polyorganosiloxanes comprennent en outre des unités de formule : 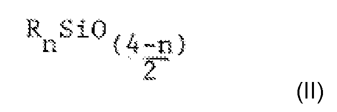 dans lequel R est un groupe tel que défini dans la revendication 11, est n est égal à 1, 2 ou 3.

12. Procédé selon la revendication 3 dans lequel le composé X est choisi parmi les oligomères ou polymères organiques, les oligomères ou polymères hybrides organique / silicone, lesdits oligomères ou polymères portant au moins 2 groupements aliphatiques insaturés réactifs et leurs mélanges.

13. Procédé selon l'une quelconque des revendications 3 à 12 dans lequel le composé Y est choisi parmi les organosiloxanes comprenant au moins deux groupes Si-H libres.

14. Procédé selon l'une quelconque des revendications 3 à 13 dans lequel le composé Y est choisi parmi les organosiloxanes comprenant au moins une unité alkylhydrogénosiloxane de formule suivante : 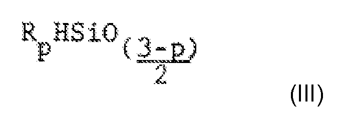 dans lequel :
- R représente un groupe hydrocarboné monovalent, linéaire ou cyclique, comprenant de 1 à 30 atomes de carbone ou un groupe phényle ; et
p est égal à 1 ou 2.

15. Procédé selon la revendication 14 dans lequel le composé Y est tel que les radicaux R représentent un groupement alkyle en C₁-C₁₀.

16. Procédé selon l'une quelconque des revendications 13 à 15 dans lequel les organosiloxanes Y comprennent au moins deux unités alkylhydrogénosiloxane de formule H₃CHSiO et comprennent éventuellement des unités (H₃C)₂SiO.

17. Procédé selon l'une quelconque des revendications 3 à 16 dans lequel le catalyseur est un catalyseur à base de platine ou d'étain.

18. Procédé selon la revendication 17 dans lequel le ou les catalyseurs représentent de 0,0001 % à 20 % en poids par rapport au poids total de la composition.

19. Procédé selon l'une quelconque des revendications 3 à 13 dans lequel le composé X est un polydiméthylsiloxane à groupements vinyliques terminaux et le composé Y est le méthylhydrogénosiloxane.

20. Procédé selon la revendication 1 ou 2 dans lequel le composé X et le composé Y sont susceptibles de réagir par condensation.

21. Procédé selon la revendication 20 dans lequel les composés X et / ou Y, identiques ou différents, sont des composés siliconés dont la chaîne principale comprend au moins deux groupes alcoxysilane et / ou au moins deux groupes silanol (Si-OH), latéraux et / ou en bout de chaîne.

22. Procédé selon la revendication 20 ou 21 dans lequel les composés X et / ou Y, identiques ou différents, comprennent de façon majoritaire des unités de formule :
R⁹ₛSiO_{(4-s)/2}, (IV)
dans lequel les R⁹ représentent indépendamment un radical choisi parmi les groupes alkyle comprenant de 1 à 6 atomes de carbone, le phenyle, les groupes alkyle fluorés, et s est égal à 0, 1, 2 ou 3.

23. Procédé selon la revendication 22 dans lequel les composés X et / ou Y, identiques ou différents, comprennent des unités de formule :
(R⁹₂SiO₂)_{f}- (V)
dans laquelle R⁹ est tel que défini dans la revendication 22, et f est tel que le polymère présente une viscosité à 25 °C allant de 0,5 à 3000 Pa.s.

24. Procédé selon l'une quelconque des revendications 20 à 23 dans lequel les polyorganosiloxanes comprennent au moins 2 groupes trialcoxysilane terminaux par molécule de polymère, lesdits groupes ayant la formule suivante :
-ZSiR¹ₓ(OR)₃₋ₓ (VI)
dans lequel :
les radicaux R représentent indépendamment un groupe méthyle, éthyle, n-propyle, isopropyle, n-butyle, sec-butyle, isobutyle ;
R¹ est un groupe méthyle ou éthyle ;
x est égal à 0 ou 1 ; et
Z est choisi parmi : les groupes hydrocarbonés divalents ne comportant pas d'insaturation éthylénique et comprenant de 2 à 18 atomes de carbone, les combinaisons de radicaux hydrocarbonés divalents et de segments siloxanes de formule : 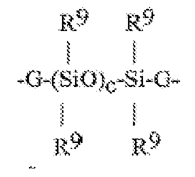
R⁹ étant tel que défini dans la revendication 22, G est un radical hydrocarboné divalent ne comportant pas d'insaturation éthylénique et comprenant de 2 à 18 atomes de carbone et c est un entier allant de 1 à 6.

25. Procédé selon l'une des revendications 20 à 24 dans lequel les polyorganosiloxanes sont choisis parmi les polymères de formule : 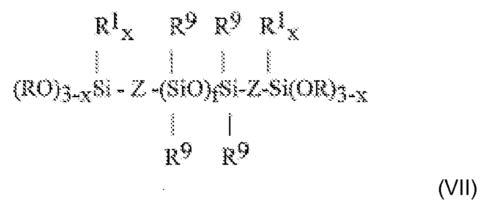 dans laquelle R, R¹, R⁹, Z, x et f sont tels que définis dans la revendication 24.

26. Procédé selon la revendication 20 dans lequel le composé X est choisi parmi les oligomères ou polymères organiques ou les oligomères et polymères hybrides organique / silicone, lesdits polymères ou oligomères comprenant au moins deux groupements alcoxysilanes et le composé Y est choisi parmi les polyorganosiloxanes.

27. Procédé selon l'une quelconque des revendications 20 à 26 dans lequel le catalyseur est un catalyseur à base de titane.

28. Procédé selon la revendication 27 dans lequel le ou les catalyseurs sont présents en une teneur allant de 0,0001 % à 20 % en poids par rapport au poids total de la composition.

29. Procédé selon l'une quelconque des revendications 20 à 28 dans lequel les composés X et Y représentent un mélange de polydiméthylsiloxanes à groupements méthoxysilanes.

30. Procédé selon la revendication 1 ou 2 dans lequel les composés X et Y sont susceptibles de réagir par réticulation en présence de peroxyde.

31. Procédé selon l'une quelconque des revendications précédentes comprenant au moins une charge choisie parmi la silice ou la silice traitée en surface.

32. Procédé selon l'une des revendications précédentes dans lequel le composé X présente une masse moléculaire en poids (Mw) allant de 150 à 1 000 000.

33. Procédé selon l'une des revendications précédentes dans lequel le composé Y présente une masse moléculaire en poids (Mw) allant de 200 à 1 000 000.

34. Procédé selon l'une des revendications précédentes dans lequel le composé X représente de 0,5 à 95 % en poids par rapport au poids total de la composition.

35. Procédé selon l'une des revendications précédentes dans lequel le composé Y représente de 0,001 à 95 % en poids par rapport au poids total de la composition.

36. Procédé selon l'une des revendications précédentes dans lequel les composés X et Y sont présents dans les compositions en un ratio X / Y allant de 0,05 à 20.

37. Procédé selon l'une quelconque des revendications précédentes dans lequel on met en oeuvre au moins un solvant organique, le ou les solvants organiques étant choisis parmi les huiles organiques ; les silicones; les huiles minérales ; les huiles végétales ; les alcanes en C₅-C₂₀ ; l'acétone ; la méthyléthylcétone ; les esters d'acides en C₁-C₂₀ liquides et d'alcools en C₁-C₈ ; le diméthoxyéthane; le diéthoxyéthane ; les alcools gras liquides en C₁₀-C₃₀ ; les esters d'alcools gras en C₁₀-C₃₀ liquides ; l'huile de polybutène ; l'isononanoate d'isononyle; le malate d'isostéaryle ; le tétra-isostéarate de pentaérythrityle ; le trimélate de tridécyle ; ou leurs mélanges.

38. Procédé selon l'une quelconque des revendications précédentes **caractérisé en ce que**, après l'application de la composition et avant l'élévation de la température des fibres capillaires au moyen d'un fer chauffant, on laisse poser ladite composition pendant un temps compris entre 30 secondes et 60 minutes.

39. Procédé selon l'une quelconque des revendications précédentes **caractérisé en ce qu'**il comprend entre l'étape d'application sur les fibres capillaires de la composition et l'étape d'élévation de la température des fibres capillaires, une étape de pré-séchage partiel des fibres capillaires.

40. Procédé selon l'une quelconque des revendications précédentes, **caractérisé par le fait que** le pH de la ou des composition(s) est compris entre 2 et 13, de préférence entre 4 et 10.

41. Procédé selon l'une quelconque des revendications précédentes, **caractérisé par le fait que** la composition contient, outre les composés X et Y, au moins un agent choisi parmi des agents réducteurs, des corps gras, des plastifiants, des adoucissants, des agents anti-mousse, des agents hydratants, des pigments, des argiles, des charges minérales, des filtres UV, des colloïdes minéraux, des peptisants, des parfums, des conservateurs, des tensio-actifs anioniques, cationiques, non ioniques ou amphotères, des polymères fixants ou non, des protéines, des vitamines, des colorants directs, des colorants d'oxydation, des agents nacrants ou opacifiants, des propulseurs, et des épaississants minéraux ou organiques tels que le benzylidène-sorbitol et les N-acylaminoacides, les cires oxyéthylénées ou non, les paraffines, les acides gras solides en C₁₀-C₃₀ tels que l'acide stéarique, l'acide laurique, les amides gras en C₁₀-C₃₀ tels que le diéthanolamide laurique, les esters d'alcool gras ou d'acides gras solides.

42. Procédé selon l'une quelconque des revendications précédentes, **caractérisé par le fait que** la composition peut se présente sous des formes diverses, telles que des lotions, des sprays, des mousses et être appliquées sous forme de shampooing ou d'après-shampooing.

43. Procédé selon l'une quelconque des revendications précédentes, **caractérisé par le fait que** l'utilisation du fer a lieu entre les applications des compositions séparées de X et Y, contenant d'une part le composé X et d'autre part le composé Y.

44. Procédé selon l'une quelconque des revendications précédentes 1 à 42, **caractérisé par le fait qu'**on utilise le fer après l'application de X et de Y.

45. Utilisation du procédé défini selon l'une quelconque des revendications précédentes pour augmenter le lissage des fibres capillaires et prolonger la durabilité du lissage.
